# EUROPEAN PATENT SPECIFICATION

(11) **EP 1 776 972 B1**
(45) Date of publication and mention of the grant of the patent: **07.04.2010**
(21) Application number: 05425741.5
(22) Date of filing: 21.10.2005
(51) Int. Cl.: A61M 1/16

(54) **Machine for dialysis with control of natraemia**
Dialysegerät mit Natraemiesteuerung
Machine de dialyse avec pilotage de la natrémie

(43) Date of publication of application: 25.04.2007
(73) Proprietor: Bellco S.r.l., Mirandola (Modena) (IT)
(72) Inventor: Baroni, Paolo, 46035 Ostiglia (IT); Cavani, Silvia, 41100 Modena (IT); Fiorenzi, Andrea, 41013 Castelfranco Emilia (IT)
(74) Representative: Jorio, Paolo

(56) References cited:
- EP-A- 0 693 297
- EP-A- 1 396 274
- US-A- 4 923 613

## Description

The present invention relates to a machine for dialysis with control of natraemia.

Dialysis is a method of purification of the blood capable of restoring the hydrosaline balance and of eliminating the water in excess and the toxic substances that accumulate in the organism as a result of renal insufficiency, transferring them to a liquid with electrolytic content similar to that of normal plasma which does not contain them. Here and in what follows, said liquid will be designated by the term "dialysing solution". Application of said method envisages that the blood, once drawn from the arm of the patient, traverses the so-called arterial line, and is introduced into the dialyser, at the outlet of which it traverses what is called venous line and returns purified to the patient.

The technique of haemodiafiltration, to which the description refers, without this implying any loss in generality, envisages that the purification of the blood will be obtained by exploiting both the phenomenon of diffusion and the phenomenon of convection. The purification by diffusion is due to a balancing of the concentrations of the blood and of the dialysing solution made to flow on opposite sides of a semi-permeable membrane, whilst purification by convection is obtained when a pressure gradient between the compartment for the dialysing solution and the compartment for the blood is set up in favour of the latter. In this way, there is a passage of plasma through the semi-permeable membrane that by entrainment also determines the passage of the toxic substances dissolved therein.

The dialysing solution does not contain those substances that it is desired to eliminate from the blood, such as urea, uric acid, creatinine, phosphorus, etc., whereas it contains a precise quantity of other substances that are to be re-balanced, such as sodium, calcium, magnesium, potassium, etc.

Sodium is the electrolyte with the highest concentration in the extracellular space and hence in the blood. It may happen that, on account of the excessive loss of sodium from the extracellular space, there will be a relative intracellular hyperosmolarity, with recall of water within the cell and cellular hyperhydration (disequilibrium syndrome). This phenomenon, together with the process of ultrafiltration, i.e., of removal of water from the vessel, reduces the capacity of the cardiocirculatory system for adapting to the smaller volume of blood circulating, thus favouring the onset of arterial hypotension. On the other hand, high sodium levels frequently lead to arterial hypertension, with a marked increase in the sensation of thirst, and to excessive interdialytic weight increase.

A solution for overcoming the problems referred to above is that of varying the levels of sodium in the dialysing solution in the course of dialysis, starting from high values during the first part of the session, then dropping progressively. This ought to enable a more gradual variation of the sodium concentration in the plasma. This variable profile of the sodium, depending upon the conditions of the patient, is obtained by programming the dialytic unit with a computer interfaced thereto.

The solution proposed above suffers from the disadvantage of not being able to take into account, and hence intervene on, possible dysfunctions regarding the levels of sodium in the blood of the patient during dialysis.

The aim of the present invention is to provide a machine for dialysis, the technical characteristics of which will be such as to solve the problems of the known art.

The subject of the present invention is a machine for dialysis comprising: a haemodialysis filter; an inlet branch for inlet of a dialysing solution in said filter; an outlet branch for outlet of the dialysing solution from said filter; an arterial line, which is responsible for transport of the blood from the patient to the filter; a venous line, which is responsible for transport of the blood from the filter to the patient; and a plurality of pumps designed for the circulation both of the blood and of the dialysing solution; said machine being characterized in that it comprises: a device for measuring the sodium concentration, applied on an ultrafiltered-plasma line; a preparing device for preparing the dialysing solution; and a control unit connected both to said device for measuring the sodium concentration and to said preparing device, said control unit being configured to adapt the sodium concentration in the dialysing solution as a function of the sodium concentration measured by the device for measuring the sodium concentration.

According to a preferred embodiment, the machine for dialysis forming the subject of the present invention comprises an isolated-ultrafiltration device set along the arterial line of the blood of the patient and designed to produce the ultrafiltered plasma, on which said device for measuring the concentration of sodium is applied.

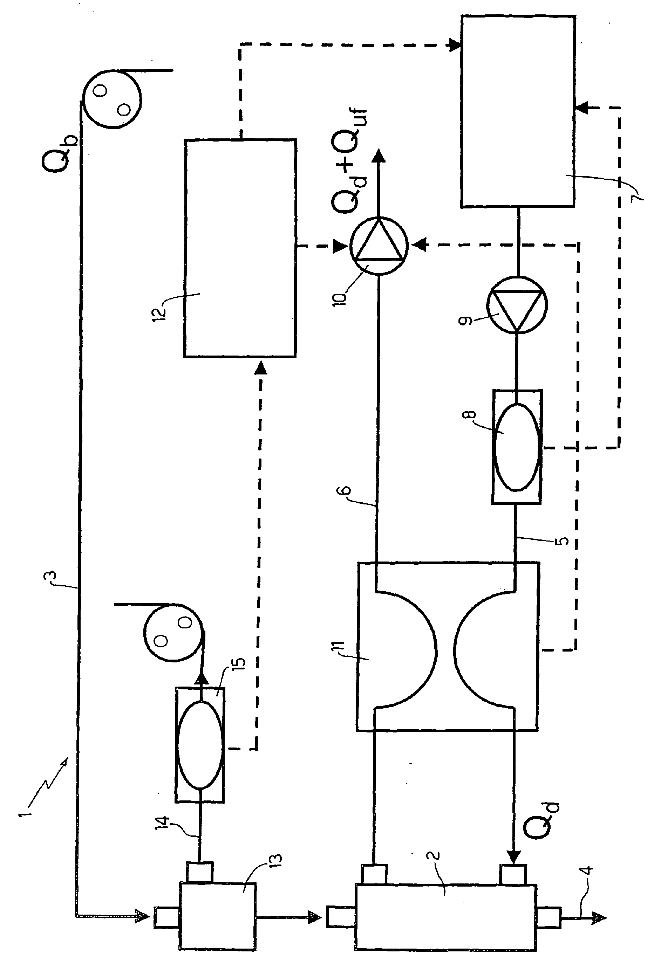

Described in what follows is an illustrative and non-limiting example in order to facilitate understanding of the invention with the aid of the figure of the annexed plate of drawings, which provides a schematic view of part of a preferred embodiment of the machine for dialysis according to the present invention.

Designated as a whole by 1 in the figure is a machine for dialysis (illustrated only partially), the components of which are represented schematically.

The machine 1 comprises: a haemodiafiltration filter 2 (known and not described in detail); an arterial line 3, which is responsible for transport of the blood from the patient to the filter 2; a venous line 4, which is responsible for transport of the blood from the filter 2 to the patient; an inlet branch 5 and an outlet branch 6 for the dialysing solution, respectively, into and out of the filter 2; a device 7 for preparing the dialysing solution, said device being connected to the inlet branch 5; a conductivity meter 8 applied to the inlet branch 5 and connected to the preparing device 7, a pair of pumps 9 and 10 applied, respectively, to the inlet branch 5 and to the outlet branch 6 for the dialysing solution; a differential flowmeter 11, described in the patent No. EP1342479 filed in the name of the present Applicant; and a control unit 12 connected to the preparing device 7, the pump 10, and the differential flowmeter 11.

The machine 1 comprises an isolated-ultrafiltration device 13 set along the arterial line 3 in such a way as to produce a quantity of ultrafiltered plasma, which is introduced into an arterial branching line 14. Generally, the arterial branching line 14 comprises a device for purification by adsorption and reconnects to the arterial line 3 in the stretch comprised between the isolated-ultrafiltration device 13 and the haemodialysis filter 2. The machine 1 further comprises a concentration meter 15 for measuring the concentration of sodium in the blood, which is applied to the arterial branching line 14 and is connected to the control unit 12.

The machine described above enables continuous reading of the level of sodium in the blood and transmission of the results to the control unit 12. The control unit 12, where algorithms are implemented for adapting the values of conductivity and concentration of sodium in the dialysing solution as a function of the level of sodium present in the blood of the patient, intervenes on the dialysing solution through the preparing device 7 and the pump 10 to re-establish, whenever the need might arise, the correct value of sodium in the blood of the patient.

As will be obvious, the machine for dialysis forming the subject of the present invention prevents the patient undergoing dialysis from possibly being subjected to clinical complications due to excessively low or excessively high levels of sodium in the blood.

Finally, the machine for dialysis according to the present invention can be adapted to any technique of dialysis, such as, for example, that of haemodiafiltration.

## Claims

1. A machine (1) for dialysis comprising: a haemodialysis filter (2); an inlet branch (5) for inlet of a dialysing solution into said filter (2); an outlet branch (6) for outlet of the dialysing solution from said filter (2); an arterial line (3), which is responsible for transport of the blood from the patient to the filter (2); a venous line, which is responsible for transport of the blood from the filter (2) to the patient, and a plurality of pumps (9, 10) designed for circulation both of the blood and of the dialysing solution; said machine for dialysis being **characterized in that** it comprises a device for measuring the sodium concentration (15) applied on an ultrafiltered-plasma line (14), a device (7) for preparing the dialysing solution, and a control unit (12) connected both to said device for measuring the sodium concentration (13) and to said preparing device (7); said control unit (12) being configured to adapt the sodium concentration in the dialysing solution as a function of the sodium concentration measured by the device for measuring the sodium concentration (15).

2. The machine for dialysis according to Claim 1, **characterized in that** it comprises an isolated-ultrafiltration device (13) set along the arterial line (3) of the blood of the patient, said device for measuring sodium concentration (15) being applied to the ultrafiltered plasma produced by said isolated-ultrafiltration device (13).

3. The machine for dialysis according to Claim 1 or Claim 2, **characterized in that** said control unit (12) is connected also to a pump (10) applied to said outlet branch (6) for the dialysing solution.

4. The machine according to any one of the preceding claims, **characterized in that** it comprises a conductivity meter (8) applied on said inlet branch (5) for the dialysing solution and connected to said preparing device (7).

## Patentansprüche

1. Eine Dialysevorrichtung (1) mit: einem Hämodialysefilter (2); einem Einlasszweig (5) zum Einlassen einer Dialyselösung in den Filter (2); einem Auslasszweig (6) zum Auslassen der Dialyselösung aus dem Filter (2); einer artiellen Leitung (3), welche verantwortlich ist für den Transport des Blutes vom Patienten zu dem Filter (2); einer venösen Leitung, welche verantwortlich ist für den Transport des Blutes vom Filter (2) zu dem Patienten, und einer Mehrzahl von Pumpen (9, 10), ausgelegt für die Zirkulation sowohl von Blut als auch von Dialyselösung; wobei die Dialysevorrichtung **dadurch gekennzeichnet ist, dass** sie umfasst: eine Einrichtung zum Messen der Natriumkonzentration (15), angebracht an einer Leitung (14) für ultrafiltriertes Plasma, eine Einrichtung (7) zum Vorbereiten der Dialyselösung und eine Steuereinrichtung (12), die sowohl mit der Einrichtung zum Messen der Natriumkonzentration (13) als auch mit der Vorbereitungseinrichtung (7) verbunden ist; wobei die Steuereinheit (12) konfiguriert ist, um die Natriumkonzentration in der Dialyselösung als eine Funktion der Natriumkonzentration, die mittels der Einrichtung zum Messen der Natriumkonzentration (15) gemessen wurde, anzupassen.

2. Die Dialysevorrichtung nach Anspruch 1, **dadurch gekennzeichnet, dass** sie eine isolierte Ultrafiltrationseinrichtung (13) umfasst, die entlang der arteriellen Leitung (3) für das Patientenblut angeordnet ist, wobei die Einrichtung zum Messen der Natriumkonzentration (15) an dem ultrafiltrierten Plasma, welches durch die isolierte Ultrafiltrationseinrichtung (13) erzeugt wird, eingesetzt wird.

3. Die Dialysevorrichtung nach Anspruch 1 oder Anspruch 2, **dadurch gekennzeichnet, dass** die Steuereinheit (12) ebenfalls mit einer Pumpe (10) verbunden ist, die an dem Auslasszweig (6) für die Dialyselösung angeordnet ist.

4. Die Dialysevorrichtung nach irgendeinem der vorhergehenden Ansprüche, **dadurch gekennzeichnet, dass** sie einen Leitfähigkeitsmesser (8) umfasst, der an dem Einlasszweig (5) für die Dialyselösung angeordnet ist und mit der Vorbereitungseinrichtung (7) verbunden ist.

## Revendications

1. Machine (1) pour dialyse comprenant : un filtre d'hémodialyse (2) ; une ramification d'entrée (5) pour l'entrée d'une solution de dialyse dans ledit filtre (2) ; une ramification de sortie (6) pour la sortie de la solution de dialyse dudit filtre (2) ; une conduite artérielle (3) qui est responsable du transport du sang du patient au filtre (2) ; un tube de veine qui est responsable du transport du sang du filtre (2) au patient et une pluralité de pompes (9, 10) conçues pour la circulation à la fois du sang et de la solution de dialyse ; ladite machine pour dialyse étant **caractérisée en ce qu'**elle comprend un dispositif pour mesurer la concentration de sodium (15) appliqué sur une conduite de plasma ultrafiltré (14), un dispositif (7) pour préparer la solution de dialyse et une unité de commande (12) raccordée à la fois audit dispositif pour mesurer la concentration de sodium (13) et audit dispositif de préparation (7) ; ladite unité de commande (12) étant configurée pour adapter la concentration de sodium dans la solution de dialyse en fonction de la concentration de sodium mesurée par le dispositif pour mesurer la concentration de sodium (15).

2. Machine pour dialyse selon la revendication 1, **caractérisée en ce qu'**elle comprend un dispositif d'ultrafiltration isolé (13) placé le long de la conduite artérielle (3) du sang du patient, ledit dispositif pour mesurer la concentration de sodium (15) étant appliqué sur le plasma ultrafiltré produit par ledit dispositif d'ultrafiltration isolé (13).

3. Machine pour dialyse selon la revendication 1 ou la revendication 2, **caractérisée en ce que** ladite unité de commande (12) est raccordée également à une pompe (10) appliquée à ladite ramification de sortie (6) pour la solution de dialyse.

4. Machine selon l'une quelconque des revendications précédentes, **caractérisée en ce qu'**elle comprend un dispositif de mesure de conductivité (8) appliqué sur ladite ramification d'entrée (5) pour la solution de dialyse et raccordé audit dispositif de préparation (7).
